(19) **Europäisches Patentamt / European Patent Office / Office européen des brevets**

(11) **EP 1 476 195 B1**

(12) **EUROPEAN PATENT SPECIFICATION**

(45) Date of publication and mention
of the grant of the patent:
**13.05.2015 Bulletin 2015/20**

(51) Int Cl.:
*A61K 47/12* (2006.01)     *A61K 47/14* (2006.01)
*A61P 25/00* (2006.01)     *A61K 31/335* (2006.01)
*A61K 33/00* (2006.01)     *A01N 63/02* (2006.01)

(21) Application number: **01959903.4**

(22) Date of filing: **19.07.2001**

(86) International application number:
**PCT/ZA2001/000099**

(87) International publication number:
**WO 2002/005851 (24.01.2002 Gazette 2002/04)**

(54) **ENHANCEMENT OF THE ACTION OF CENTRAL AND PERIPHERAL NERVOUS SYSTEM AGENTS**

VERBESSERUNG DER WIRKUNG VON ZENTRALEN UND PERIPHEREN
NERVENSYSTEMMITTELN

RENFORCEMENT DE L'ACTION D'AGENTS SUR LE SYSTEME NERVEUX CENTRAL ET
PERIPHERIQUE

(84) Designated Contracting States:
**AT BE CH CY DE DK ES FI FR GB GR IE IT LI LU
MC NL PT SE TR**

(30) Priority: **19.07.2000 ZA 200003643**

(43) Date of publication of application:
**17.11.2004 Bulletin 2004/47**

(73) Proprietor: **North-West University
Potchefstroom 2520 (ZA)**

(72) Inventor: **MEYER, Petrus, Johannes
6573 Sedgefield (ZA)**

(74) Representative: **Bates, Philip Ian et al
Reddie & Grose LLP
16 Theobalds Road
London WC1X 8PL (GB)**

(56) References cited:
**WO-A-93/25213     WO-A-97/17978**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD OF THE INVENTION

This invention relates to pharmaceutical preparations (which expression is herein intended to include veterinary preparations) for use in treating afflictions of the animal body (which expression is herein intended to include the human body) affecting the central and/or peripheral nervous system of an animal in need of treatment.

### BACKGROUND TO THE INVENTION

In EP 93912877.3 and US patent 5,633,284 and their equivalents the applicant disclosed that dermatological or topical compositions comprising the combination of nitrous oxide [$N_2O$] and at least one fatty acid, or lower alkyl ester thereof, in a dermatologically acceptable carrier medium, are useful in the treatment of a variety of skin, muscle and joint disorders. It further disclosed therein that such combinations may beneficially also include additional active ingredients. The following active ingredients are specifically mentioned in this regard: coal tar solution, collagen, nicotinamide, nicotinic acid, lanolin, vitamin E, methyl salicylate, arnica and H1-antagonist antihistamines of which only diphenhydramine chloride is specifically mentioned.

No agent known for having an effect on the central or peripheral nervous system, was amongst the active ingredients specifically mentioned in these patents. Mention was made therein that coal tar solution (also known as *Liquor Picis Carbonis*) may be used as a supplementary active ingredient and that the resultant preparation is suitable for use in the treatment, *inter alia*, of psoriasis, eczemaceous conditions and dermatitis. It is also disclosed in these patents that, in addition to the coal tar solution the composition may also contain an H1-antagonist antihistamine (e.g. diphenhydramine hydrochloride) and that such a preparation may beneficially be used in the treatment of atopic and allergic conditions manifesting in skin irritations such as eczema and dermatitis. The aforementioned conditions have since the date of the above patents come to be regarded by some writers on the topic as manifestations in the skin of underlying disorders which have or include a neurological, or immunological, or some even suggest a neuro-immunological cause. These patents however do not refer to that explanation. It further gives no hint that the effects obtained are the result of any form of systemic operation of any of the components of the formulation. Coal tar liquid is not known to be an agent acting on the central nervous system. Insofar as these patents may be seen to proffer any explanation at all for the results obtained, which is not what the applicant contends for, it is suggested that such explanation would, within the context of the disclosures therein, point to a local effect of soothing irritation and of repairing of skin and associated tissue damage as a result of the conditions mentioned therein. These are psoriasis, shingles, fever blisters, chicken pox, acne, chilblains, eczema, chloasmas, alopecia, dermatitis, ringworm and burn wounds.

In WO97/17978 and US patent 6,221,377 and their corresponding applications and patents the present applicant disclosed that the action of analgesic, anti-inflammatory and anti-pyretic drugs may be enhanced by administering such drugs in conjunction with a medium which comprises nitrous oxide and at least one long chain fatty acid selected from the group consisting of oleic acid, linoleic acid, alpha-linolenic acid, gamma linolenic acid, arachidonic acid, and any of the $C_1$ to $C_6$ alkyl esters of such long chain fatty acids, mixtures of such acids and mixtures of such esters. The medium may comprise the mixture known as Vitamin F Ethyl Ester and may optionally further comprise eicosapentaenoic acid [C20:5ω3] and decosahexaenoic acid [C22:6ω3].

While these applications do disclose a systemic effect in that the formulations are, for example, topically applied or orally administered to have an effect, say, in an affected joint or muscle, the specification again fails to disclose the mode or route of transport of the active ingredient from its site of administration to its locus of effect, and speculation in this regard would probably be based on *ex post facto* hindsight.

It has now surprisingly been found that the aforesaid medium has the unexpected property that it displays a remarkable ability to enhance the action of known agents affecting the central and/or peripheral nervous system, such known agents being other than the group which consists of coal tar solution, H1 antagonist antihistamines, the analgesics, the antiinflammatories and the antipyretics. These agents will hereinafter collectively be referred to as "CPNS agents" which term is for purposes of this specification intended to embrace those biologically active compounds which perform their action on the central or peripheral nervous system of the human or animal body, but to exclude recognised anti-inflammatory, analgesic and antipyretic substances and also excluding coal tar solution and H1 agonist antihistamines. Subject to these exclusions it therefore includes the compounds which fall within Class 1 of the Pharmacological Classification set out as part of Regulation 5(1) of the General Regulations made under The Medicines and Related Substances Control Act, Act 101 of 1965, and the active ingredients of all products falling within Class 1 of the pharmacological classification currently employed in the Monthly Index of Medical Specialities ("MIMS") published by Times Media in South Africa, but excluding the analgesic, anti-inflammatory and anti-pyretic compounds which falls within the above two partially overlapping classes. Examples of some of the compounds falling within the group is described in greater detail below.

The exclusion of coal tar solution and H1-antagonist antihistamines, and of anti-inflammatories, analgesics and antipyretics from the ambit of the present invention is introduced without thereby conceding that the aforementioned patents and applications contain any disclosure of any CPNS agent properties of such excluded compounds, or that such properties are obvious in the light of the disclosures in such patents or applications. Such inferences are specifically denied. The exclusion is introduced simply to avoid what is anticipated to be a potential obstacle to the grant of a patent in respect of a part of the subject matter which part in itself is not considered worth contesting during examination as it might unduly delay the implementation in practice of the significant features of the present invention. It is expected that the remaining bulk of the subject matter of the present invention will greatly contribute to the accessibility of medicines for the treatment of a large range of ailments.

It has been pointed out in WO97/17978 referred to above that nitrous oxide is a natural gas which is also produced synthetically, that it is also known by the trivial name "laughing gas", and that it has been in use for many years as an inhalation anaesthetic and analgesic, particularly in dentistry.

It was further stated that nitrous oxide has been reported to have a synergistic or potentiating effect on halothane and other gaseous anaesthetics [See Goodman & Gilman's The Pharmacological Basis of Therapeutics 8th Ed. 1990 pp. 298-300].

Since such known synergism or potentiation is based on the use of nitrous oxide administered by inhalation, and since the use of nitrous oxide on its own as an anaesthetic and analgesic has likewise been in the form of an inhalation agent, the use of nitrous oxide for all these purposes has been confined to hospitalised patients or, at best, to treatments carried out by medical practitioners in their consulting rooms, or treatments carried out by or under supervision of a nurse in charge of a home-care patient.

Nitrous oxide is known to be soluble in water and it has been reported that at 20°C and 2 atm pressure one litre of the gas dissolves in 1,5 litres of water, see The Merck Index 10th Ed. p. 6499.

Nitrous oxide is also known for its use as a propellant gas, mainly as a substitute for propellant gases such as chlorofluorocarbons, and more particularly to produce a food product mousse such as whipped cream or chocolate mousse or quick-breaking foams for hair treatment preparations. See In this regard U.K. Patent 1033299, U.K. Patent 1105919 and European Patent Application EPA-0123827. None of these prior publications suggests that the nitrous oxide gas plays any other role than a physical one, i.e. to expand on being depressurised and thereby to create a mousse or a foam. In fact It is typically regarded as an inert in these applications and useful due to the fact that it is colourless, odourless and tasteless but soluble in water and oils.

There appears to be no suggestion In the literature, other than the applicant's own prior patents and patent applications referred to above, that aqueous solutions of nitrous oxide might have any effect on man or animals. As far as the present inventors know It has also never been suggested that nitrous oxide may be used in conjunction with the CPNS to enhance the action of such agent or to serve as a carrier for such agent.

It is known in the pharmaceutical field to formulate active ingredients in so-called liposomal formulations. Unlike the present invention which is based on formulations containing long chain fatty acids and esters thereof the liposomes are based on a clearly distinguishable group of compounds namely the phospholipids, and generally also contain cholesterol as a stabilising agent and may further contain lisolecithin. These compounds or classes form no part of the present invention and, In case it is necessary to do so, are specifically excluded from the group of long chain fatty acids and derivatives thereof incorporated in the method or formulation of the invention.

## OBJECT OF THE INVENTION

It is an object of the present invention to provide a method of enhancing the action of pharmaceutical agents selected from the psycho analeptlcs (antidepressants) and to provide pharmaceutical preparations of such agents which preparations have enhanced action compared to the action of known formulations containing the same agents.

## STATEMENTS OF THE INVENTION

According to the present invention there is provided a pharmaceutical preparation comprising Doxepin formulated with an administration medium which is characterised in that it comprises a solution of nitrous oxide gas in a pharmaceutically acceptable carrier solvent for the gas and which administration medium includes at least one fatty acid or ester or other suitable derivative thereof selected from the group consisting of oleic acid, linoleic acid, alpha-linolenic acid, gamma-linolenic acid, arachidonic acid, eicosapentaenoic acid [C20: 5ω3], decosahexaenoic acid [C22: 6ω3], ricinoleic acid and derivatives thereof selected from the group consisting of the C1 to C6 alkyl esters thereof, the glycerol-polyethylene glycol esters thereof and the reaction product of hydrogenated natural oils composed largely of ricinoleic acid based oils such as castor oil with ethylene oxide.

The administration medium may further include eicosapentaenoic acid [C20:5ω3] and/or decosahexaenoic acid [C2Z:6ω3] as additional long chain fatty acids.

The carrier solvent for the nitrous oxide gas may be water or any of the pharmaceutically acceptable alcohols, ethers, oils or polymers such as, for example a polyethylene glycol. The oll may be organic or mineral oil. The organic oil may be an essential oil based on long chain fatty acids having between 14 and 22 carbon atoms in the fatty acid. The oil may also be of either natural or synthetic origin and, if of natural origin, it may be either plant oil or animal oil. As plant oils those rich in gamma linolenic acid [GLA] are preferred and as animal oil dairy cream may be used.

In the preferred form of the invention the solution is an aqueous solution saturated with nitrous oxide.

The water is preferably de-ionised water and free of microbes.

When the Doxepin to be enhanced by means of the nitrous oxide is in a liquid formulation, such formulation may incorporate as part of the administration medium water or acceptable other liquid solvent into which the nitrous oxide and fatty acid or ester thereof had been dissolved or suspended or emulsified along with the Doxepin to be enhanced by being formulated therewith. When the formulation containing the Doxepin to be enhanced by means of the nitrous oxide is to be In a liquid (including an encapsulated liquid) presentation for oral administration or in a nasal or bronchial or pulmonary spray or in the form of an injectable formulation, such formulation may incorporate, as part of the administration medium, water or acceptable other liquid into which the nitrous oxide is dissolved and in which the fatty acid or ester thereof is either dissolved or suspended or emulsified along with the Doxepin to be enhanced by being formulated therewith.

Likewise, where the Doxepln is to be administered to the patient by being applied as a topical, buccal or vaginal cream or ointment, or as a cutaneous patch, or In the form of micro-depots, or as an intravenous, intramuscular or subcutaneous injection, or as a suppository, the formulation used in making up such cream, ointment, patch, depots or injectable formulation or suppository may incorporate, along with the Doxepin to be enhanced, a quantity of water or other liquid containing, and preferably saturated with, nitrous oxide, the long chain fatty acid or ester thereof and a agent conjugated therewith, and, further, such additional excipients and carriers as are conventionally used in the pharmaceutical trade in making up such dosage forms.

The carrier solvent for the nitrous oxide gas may thus be essentially non-aqueous and composed of the at least one fatty acid or ester thereof selected from the group consisting of oleic acid, linoleic acid, alpha-linolenic acid, gamma-linolenic acid, arachidonic acid, eicosapentaenoic acid [C20: 5$\omega$3], decosahexaenoic acid [C22: 6$\omega$], ricinoleic acid and derivatives thereof selected from the group consisting of the C1 to C6 alkyl esters thereof, the glycerol-polyethylene glycol esters thereof and the reaction product of hydrogenated natural oils composed largely of ricinoleic acid based oils with ethylene oxide, required to be part of the formulation.

The essential fatty acid, or ester thereof, component of the composition preferably comprises a mixture of esters of the fatty acids listed above. Thus, in the most preferred form of the invention the fatty acid component of the composition is constituted by the complex known as Vitamin F and in this regard it is preferred to make use of the ester form of Vitamin F known as Vitamin F Ethyl Ester. This product is commercially available under the trade description of Vitamin F Ethyl Ester CLR 110 000 Sh.L. U./g from CLR Chemicals Laboratorium Dr.Kurt Richter GmbH of Berlin, Germany. The typical fatty acid distribution of this product is as follows:

| | |
|---|---|
| $<C_{16}$ : | 0 |
| $C_{16.0}$ : | 8,3% |
| $C_{18.0}$ : | 3,5% |
| $C_{18.1}$ : | 21,7% |
| $C_{18.2}$ : | 34,8% |
| $C_{18,4}$ : | 28,0% |
| $>C_{18}$ : | 1,6% |
| unknown: | 2,1% |

It is further preferred to add to the formulation the long chain fatty acids known as eicosapentaenoic acid [C20:5$\omega$3] and decosahexaenoic acid [C22:6$\omega$3]. Such a product combination is available from Roche Lipid Technology under the trade name "Ropufa '30' n-3 oil".

It has been found by microscopic studies that the formulation of active agents with a medium as herein described gives rise to the formation of minute, generally spherical bodies, in which the active ingredient is contained in a stable form and from which it is delivered at the site of action.

An agent of specific relevance in this application is doxepin hydrochloride. Doxepin HCl is one of a class of psychotherapeutic agents known as dibenzoxepin tricyclic compounds. Specifically, it is an isomeric mixture of 1-Propanamine,3-dibenz[b,e]oxepin-1,1(6H)ylidene N,N-dimethyl-hydrochloride. In oral formulation it is useful as an antidepressant to relieve symptoms of depression and anxiety such as feelings of sadness, worthlessness, or guilt; loss of interest in daily activities; changes in appetite; tiredness; sleeping too much; insomnia, and thoughts of death or suicide.

Doxepln Is also sometimes used to treat certain types of pain but it is not generally regarded as an analgesic in the ordinary sense of the word and has been described as one of a group of "adjuvant analgesics" along with other anti-depressants. In a topical formulation doxepin is known to be used as an anti-pruritic to relieve itching in patients with certain types of eczema. The applicant is unaware of any prior disclosure suggesting that doxepin may beneficially be used in a topical administration form for alleviating pain.

The applicant found that a potent topical formulation may be prepared according to the invention by formulating Doxepin with the medium as described.

It is accordingly an aspect of the present invention to provide a topical formulation, used either as a lotion or a cutaneous patch as described above in which doxepin is the active ingredient.

## PRELIMINARY HYPOTHESES OF MECHANISM OF OPERATION

The mechanism by which the enhancement of action of CPNS drugs is achieved by the present invention, is currently under investigation. Some observations in this regard have been recorded above. In addition it Is recorded that preliminary observations point to some additional possible explanations. The applicant again does not wish to be bound to any of the tentative explanations it may put forward at this time. It is recorded, however, that it would appear that the long chain fatty acids used in the formulation of the preparation according to the invention, or at least some of these components, form, during the manufacturing process of the medicinal formulation, very small spherical bodies, hereinafter referred to as "nanolipid vesicles". These nanolipid vesicles have dynamic characteristics in respect of the encapsulation and subsequent delivery of compounds at predicted areas in cells and organisms where the optimal utilisation of these compounds occur with resultant maximised modes of actions.

The present model for the understanding of the invention is that the dynamic delivery characteristics of the nanolipid vesicles are utilised efficiently to transport compounds to locations where maintenance of optimal concentrations in the organisms or cells is beneficial in combating specific infective diseases. The cells in this application most probably concern neurons. The long chain fatty acids may contribute to the maintenance of the myelin sheath of the nervous system, being precursors of the sphingomellin molecules.

These beneficial effects are believed to be attributable to the dynamic characteristics of the nanolipid vesicles. The current hypothesis is that these characteristics include:

## 1. The structural characteristics of the formulation of the preparation:

Nitrous oxide and the unsaturated long chain fatty acids forming part of the administration medium are formulated by being mixed with designated CPNS agents or compounds to form the nanolipid vesicles containing the compound or CPNS agent Two important observations have been made in this regard:

a) It was found that when the unsaturated long chain fatty acids used are 20 carbons or more, the nanolipid vesicles form spherical structures with sub-compartments similar to those seen in a sponge.
These structures are stable and it is our belief that antibodies or other ligands would fit ideally in these sub-compartments so that the nanolipid vesicles bind to specific epitopes or receptors at the target cell surface.

b) When unsaturated long chain fatty acids of 16 to 20 carbons are used, the form of the nanolipid vesicles is spherical with a dynamic field of moving autofluorescent particles surrounding the vesicles.
When nitrous oxide is omitted from the process the moving particles surrounding the nanolipid vesicles move erratically and asymmetrical movements are then detected.
It is believed that nitrous oxide is essential in stabilising the moving autofluorescent particles surrounding the nanolipid- vesicles, which is an essential characteristic to efficient compound delivery.

## 2. Stability:

The nanolipid vesicles appear to remain structurally intact after 24 months at room temperature. Any encapsulated active compounds remain encapsulated during this time. This stability feature is believed to be of substantial significance and one of the contributing factors for the enhancement observed.

## 3. Absence of cytotoxicity:

The nanolipid vesicles have no apparent cytotoxicity. When applied to cells in culture, at applicable concentrations they appear rather to have a beneficial effect on normal cell growth.

**4. Targeting:**

It has been observed that the nanolipid vesicles with unsaturated long chain fatty acids of 16 to 20 carbons, when added to cells in culture, concentrate around the mitochondria which are In the region of the nuclear membrane. Nanolipid vesicles appear to display a natural affinity towards mitochondria. Furthermore mitochondria contain a specific transport mechanism for long chain fatty acids.

When the unsaturated fatty acids used are longer than 20 carbons, it is thought that the nanolipid vesicles target peroxisomes. It Is known that peroxisomes metabolise fatty acids containing more than 20 carbons.

In *in vivo* applications, the biodisposition of nanolipid vesicle-encapsulated compounds has been found to favour the liver and spleen. Although the transport of normally readily diffusible compounds across the blood brain barrier is inhibited by the nanolipid vesicles, the nanolipid vesicles have been shown to deliver and release active compounds through the blood brain barrier to a greater extent than current commercial preparations, as will be shown for Bupicvicaine (see Example). The nonolipid vesicles may furthermore contribute to the bloavailability of orally administered conjugations of an active compound and the nanolipid vesicles, as was shown for antibiotic compounds, which is the subject of a separate patent application. In addition, the nanolipid vesicle may contribute to the protection or stabilization of the active compound in the blood, especially in the case of peptides being used as active compound, where the compound is prone to digestion by proteases.

**5. Mechanism of action:**

5.1 **Loading efficiency:**

The high loading efficiency of nanolipid vesicles has been demonstrated by achieving a high degree of encapsulation of a wide range of active drugs.

5.2 **Transport:**

The nanolipid vesicles behave as a transport mechanism to carry molecules such as active compounds.

5.3 **Release:**

It has been shown that the nanolipid vesicles have very high delivery efficiencies. The high delivery efficiency relates to tissue penetration, cell adsorption, internalisation of nanolipid vesicles by cells, parasites and bacteria, intracellular stability, and subsequent sub-cellular organelle delivery.

The result of high delivery efficiency is the release of active compounds not only at membrane sites, but also at intracellular sites including the nucleus of viable cells or microorganisms. The result is an enhanced efficacy of said active compound. The nanolipid vesicles and active compound appear to act synergistically in attaining enhanced efficacy.

**6. Elasticity:**

Confocal laser scanning microscopy (CLSM) shows that the conformation of nanolipid vesicles can be changed while in movement.

When the vesicles move through membranes, the conformation changes so that the intracellular nanolipid vesicles may have other morphological characteristics.

While moving through membranes the nanolipid vesicles 'feed' the membranes with unsaturated long chain fatty acids which in its turn will have a positive effect on membrane bound processes. This process has a positive effect on the metabolism of the cell and thus the survival of the cells.

**7. Dynamic inter-lipid vesicle relationships:**

It has been shown that vesicle inter-lipid relationships do exist. The lipid vesicles can interchange the compounds they respectively carry.

They can also combine to resize themselves continuously without detriment to their stability. The inter-lipid relationship is also revealed when moving through the cellular membrane.

These interactive membrane characteristics make the movement of the vesicles through the cells optimal.

Although inter-relationships of the dynamic nanolipid vesicles are continuously present, it has also been shown that the particles are stable in blood and body fluids for up to 5 hours.

## EXAMPLES OF THE INVENTION

Without thereby limiting the scope of the invention some examples will now be described to illustrate the invention. Two preparations which do not form part of the claimed subject matter of this application, being preparations first disclosed in the applicant's own prior patents referred to above, are first restated.

## PREPARATION 1

### Preparing an aqueous nitrous oxide solution

A pressure vessel is charged to its operating volume with water at 20°C [ambient temperature]. The vessel is connected to a supply of nitrous oxide via a flow control valve and pressure regulator. The closed vessel is supplied with nitrous oxide at a pressure of 2 bar for a period of 48 hours, it having been determined that at the aforementioned temperature the water is saturated with nitrous oxide over such period of time under the above-mentioned pressure.
A resultant solution is bottled as stock solution for use in the formulations and applications set out below.

## Comparative PREPARATION 2

### Preparation of an aqueous nitrous oxide/Vitamin F emulsion

30g Vitamin F ethyl ester as identified and described above was mixed with 10g chremaphor, 2,2g methyl paraben, 0,08g butyl hydroxyanisole, 0,23g butyl hydroxytoluene with stirring at 80°C.
Into 942,5g of the stock nitrous oxide solution was dissolved 2,5g sodium propyl paraben and 2,5g Germall 115 [Imidurea] with stirring at room temperature.
The oily composition first described was emulsified into the aqueous solution with stirring to constitute a stock nitrous oxide/Vitamin F emulsion. It is herein referred to as the "nanolipid-vesicle" formulation.

## Comparative Preparation 3

### Injectable solution of active in an injectable solution, containing nitrous oxide in carrier formulation.

The manufacturing process for producing an injectable formulation of an active agent according to the invention will now be described with reference to the manufacture of a medicament containing Bupivacaine as active ingredient
**Preparation X** was made up according to the following protocol:

*Step 1:* Weigh off the 546g of Bupivicaine HCl and add to the pot. Mix thoroughly with $N_2O$-saturated water until the active compound is completely dissolved.

*Step 2:* Weigh off and add together the Poloxyl hydrogenated castor oil 1,023Kg, Vitamin F ethyl ester 3.012Kg, into mixing pot 2 and heat to approximately 70°C until all the oil has melted.

*Step 3:* Weigh off and add the Methyl paraben 214g and the Butylated hydroxytoluene 36g to mixing pot 2, in the respective order, while continuously mixing ensuring each solid is dissolved before adding the next while still maintaining the temperature at 70°C.

*Step 4:* Combine the contents of Step 1 and Step 3 whilst stirring during addition and continue stirring until the phases are well and truly mixed. Determine and adjust the pH to near physiological pH if necessary.

*Step 5:* Sonicate and check vesicle size.

*Step 6:* Sterilise by filtration and pack into amber glass ampoules or vials.

## Comparative Preparation 4

### Preparation of a typical formulation having a non-aqueous solution of nitrous oxide in carrier formulation.

The manufacturing process for producing a non-aqueous formulation of an CPNS agent according to the invention is based on the principles and ratios of ingredient described with reference to the manufacture of a medicament containing for example Phenythoin Na as active ingredient

**Preparation Y** was made up based on the following protocol:

**Step 1:** Weigh of the applicable weight of Phenytoin Na for the purpose required, i.e. age of patients, severity of condition. Reduce the particle size to less than $60\mu m$.

**Step 2:** Weigh off and add together the Poloxyl hydrogenated castor oil to a final concentration of 11.5% (w/w) Vitamin F ethyl ester to a final concentration of 23.5% (w/w) and Polyethylene glycol 400 to a final concentration of 12% (w/w) into mixing pot 2 and heat to approximately 40°C until all the oil has melted. dl-$\alpha$-Tocopherol may be added depending on the physico-chemical characteristics of the active compound, the rate of release and period of release needed, Eicosapentaenoic acid and/or decosahexaenoic acid may be added at this step.

**Step 3:** Gas the oil mixture with nitrous oxide for 3 hours at 2 bar in the stainless steel pressure vessel in the manner described in Preparation 1 above.

**Step 4:** Transfer the gased mixture to mixing pot 1 and heat to approximately 70°C .

**Step 5:** Weigh off and add the Methyl paraben to a final volume of 0.5% and the Butylated hydroxytoluene 0.05% to mixing pot 1, while continuously mixing ensuring each solid is dissolved before adding the next while still maintaining the temperature at 70°C.

**Step 6:** Remove from the heat and allow to cool down to approximately 40°C.

**Step 7:** Add the required amount of Phenytoin Na stepwise while continuously mixing.

**Step 8:** Gas the mixture from step 7 with nitrous oxide at 20kPa for 30 minutes with mixing until the mixture has reached room temperature.

The Phenytoin Na Preparation prepared as set out above was encapsulated in soft gel capsules in the manner well known in the pharmaceutical trade as oral capsules for use as described below.

## EXAMPLE 1

### PREPARATION OF TOPICAL FORMULATIONS OF DOXEPIN HYDROCHLORIDE, LIDOCAINE HYDROCHLORIDE, AND SCOPOLAMINE HYDROCHLORIDE IN THE NANOLIPID VESICLE FORMULATION OF PREPARATION 2

(a) A 2% composition of doxepin in the nanolipid-vesicle composition of Preparation 2 above was prepared by mixing doxepin hydrochloride with the nanolipid vesicle formulation as described in Preparation 2 which had been modified by the addition of Keltrol to render a thick creamy consistency, in a mass/mass ratio of 2:98 with the aid of a high speed stirrer. The composition is designated DOXIPEX CREAM.

(b) In similar manner, to be used for comparision, a 1.7307% composition of Lidocaine Hydrochloride was prepared and designated LIDAREX LOTION.

(c) A 0.3% composition of Scopolamine HBR, for use as comparision, was also prepared in the same manner and designated MZL Scopolamine.

(d) A 0.5 nasal spray composition of Oxymetazoline HCl, for use as comparision, was also prepared in the same manner with some modification and designated Oxymetazoline MZL.

(e) A 0.5% injection formulation of Bupivicaine HCl, for use as comparision, was further prepared in the same manner but with some modification and designated Bupivicaine MZL.

**EXAMPLE 2**

**STUDY TO INVESTIGATE THE RELEASE CHARACTERISTICS OF THE FORMULATIONS PREPARED IN TERMS OF EXAMPLE 1 AGAINST COMMERCIAL PREPARATIONS**

**1. OBJECTIVE:**

The scope of the study was to establish whether the Test active agents viz. Doxepin Hydrochloride,
Lidocaine Hydrochloride (as comparative example) Scopolamine Hydrobromide (as comparative example) Oxymetazoline HCl and (as comparative example) Bupivicaine HCl (as comparative example) are released from the dosage forms prepared in accordance with the invention as described in Example 1, and to determine the extent and rate of such release. It was further done to compare the rate of release of Doxipex to that of an FDA approved 5% Doxepin Hydrochloride topical formulation which is commercially available in Canada where it is indicated for the treatment of pruritis associated with eczema. That product is herein referred to as DOXEPIN COM. The test further compares the release of Lidocaine Hydrochloride from LIDAREX against a commercially available Lidocaise Hydrochloride composition herein referred to as LIDOCAINE COM. The test further compared the release of the Oxymetazoline MZL to a commercially available oxymetazoline nasal spray formulation designated oxymetazoline Com. Finally the test compares the release of the Bupivicaine MZL formulation with a commercially available Bupivicaine injection formulation designated Bupivicaine Com.

The applicability of the method used to test for release out of the dosage forms is confirmed in *Handbook of Dissolution Testing; Dissolution Testing of Transdermal Delivery Systems, page 61.* The small receptor volume to be used, in this case 12 ml, is confirmed in the same reference.

**2. METHOD**

The in vitro release from the dosage forms was determined by a Hanson Model 57-6M, Manual Start-Up, Diffusion Cell Test System available from Hanson Research with the following main parts:

CELL DRIVE CONTROL

6-CELL DRIVE WITH CELLS

VERTICAL CELLS

**3. ADDITIONAL EQUIPMENT USED**

1. Diffusion cell assembly, including donor top and receptor chamber (set of 6). The donor top includes a drug dosage wafer (Teflon washer), an acrylic top plate, and a clamp to connect top to bottom.
2. Pig skin, mucous membrane or Dura mater as applicable, used within 24 hours from being slaughtered kept in Ringer Solution between 2°C - 8°C.
3. Davies Gold Series Dermatome, Simplex GS 102.
4. Application squeegee and tweezers.
5. Drug dosage form.
6. Absorbent paper towels and tissues.

**4. TECHNIQUE**

The technique used included the following steps:

1. Obtain skin from pig heads (jawbone skin). Use Dermatome according to the Operation standard operating procedure for the Dermatome, setting it to size the skin to a thickness of 0.33mm. The diameter of the skin should be in excess of the drug dosage wafer
**OR**
For investigations using the active compound Oxymethalzoline MZL nasal spray, obtain the nasal mucous membrane of the pig in the following manner: the snout of the pig is removed just below the eyes with a stainless steel saw blade. The snout is cut in half between the nostrils. The cartilage is then gently cut and the nasal passage opened. The mucous membrane is carefully loosened and removed. Mucous membrane cells of 2.5cm by 2.5 cm are cut. The membrane thickness is monitored with a micrometer

**OR**

For investigations using the active compound Bupivicaine MZL Injectable Solution, obtain the Dura mater (the tough membrane forming the barrier between the brain and cranium or blood) in the following manner: Open the cranium with a stainless steel saw blade. Divide the brain in half and remove carefully from the opened cranium without disturbing the membrane. Loosen the membrane from the cranium and measure the width of the membrane with a micrometer. The thickness of the membrane should be between 0.19 and 0.24 mm. Cut at least 6 cells of 2.5 by 2.5 cm of membrane.

Prepare receptor chamber of diffusion cells with slight overflow of medium as specified in table below with temperature controlled at 32°C.

2. Prepare each piece of skin, mucous membrane or Dura mater with the relevant products one at a time as follows:

2.1. Lift skin, mucous membrane or Dura mater with tweezers, place on tissue and blot excess of solution, invert and blot.

3.2 Place skin, mucous membrane or Dura mater in centered position on drug dosage wafer.

3.3 3.3 Place relevant products on top of skin, mucous membrane or Dura mater in dosage wafer cavity.

3.4 Use squeegee to carefully smooth product over membrane, filling entire cavity.

3.5 Wipe excess dosage off wafer..

3.6 Lift loaded dosage wafer with skin, mucous membrane or Dura mater and place on top of receptor cell with skin, mucous membrane or Dura mater side towards cell medium. Exclude bubbles during process. Place top plate on top of donor cell assembly, pressing down with finger,

squeezing out bubbles between top plate and dosage form. Apply clamp to lock down top donor and bottom receptor halves of diffusion cell.

## 5. OPERATION OF APPARATUS:

The apparatus must be set to 150 rpm. Samples of 150μl are withdrawn with a micropipette at 10, 20, 30, 60, 90 and 120 minutes. The samples after being withdrawn are analysed for the active compound by means of HPLC, using the parameter indicated in table below for each of the different compounds.

|  | Doxipex And Doxepin Com | Lidarex And Lidocaine Com | Scopolamine MZL | Oxymetazoline HCl MZL and Oxymetazoline HCl | Bupivicaine MZL and Bupivicaine Com |
|---|---|---|---|---|---|
| Injection volume | 20μl | 20μl | 20μl | 20μl | 20μl |
| Column | Zorbax C18 15 cm | Zorbax C18 250mm x 4.6mm | Zorbax SB C18 150mm x 4.6mm | Zorbax C18 250mm x 4.6mm | Zorbax C18 250mm x 4.6mm |
| Mobile Phase | 40% 0.2M $NaH_2PO_4$ buffer with 60% MeOH adj pH to 2.5 with $H_3PO_4$ | 50ml GAA in 930 m $H_2O$ at pH 3.4 adj with 1N NaOH | 70% Phosphate buffer: 30% MeOH, pH2.5 | 70% Phosphate buffer: 30% MeOH, pH3.5 | 65% Acetonitril e: 35% Phosphate Buffer, pH 7.7 |
| Receptor chamber medium | PH 6.8 phosphate buffer | 40% MeOH/60% $H_2O$ | PH6.8 phosphate buffer | PH 6.8 phosphate buffer | PH 6.8 phosphate buffer |

(continued)

| | Doxipex And Doxepin Com | Lidarex And Lidocaine Com | Scopolamine MZL | Oxymetazoline HCl MZL and Oxymetazoline HCl | Bupivicaine MZL and Bupivicaine Com |
|---|---|---|---|---|---|
| Detector | HPLC at 254nm | HPLC at 254 nm | HPLC at 240 nm | HPLC at 280 nm | HPLC at 263 nm |
| Temperature | Ambient (22°C) | Ambient (22°C) | Ambient (22°C) | Ambient (22°C) | Ambient (22°C) |
| Flow Rate | 1.5ml/min | 1.5ml/ min | 1.5ml/ min | 1 ml /min | 2ml/min |
| Retention Time | 3 - 4 min | 5 - 6 min | 2.5 - 2.7 min | 4.85 -5.07 min | 4.4 - 4.6 min |
| Solvent | 80% 0.2M $NaH_2PO_4$ buffer with 20% MeOH adj pH to 6.8 with 1N NaOH | MeOH/60% $H_2O$ | $KH_2PO_4$ buffer pH 6.8 | $KH_2PO_4$ buffer pH 6.8 | $KH_2PO_4$ buffer pH 6.8 |
| Cells used | 6 | 6 | 6 | 3 | 3 |
| Time at which total release determined (Min) | 60 | 30 | 15 | 8 | 5 |

## 6. RESULTS

The release experiment was performed in 6 cells for each product and the mean release is reported for each analysis point. The results were tabulated and graphically presented. The results as a percentage of the active ingredient released per label claim per cell at the different time intervals was also tabulated.

Table 1 below gives a summary of the release rates and percentage release per label claim for the products determined according to calculations, reporting the mean values at 60 minutes (1 hour) for each of these compounds.

**Table 1. Release rates and percentage release per label claim for products tested.**

| Active Agent | % Active /product | Release Rate ($\mu$g/cm$^2$/h) | % Release per label claim |
|---|---|---|---|
| Doxipex | 2.00 | 112.9951 | 0.7945 |
| Doxepin Com | 5.00 | 45.6479 | 0.3190 |
| Lidarex | 1.7307 | 0.6954 | 0.0769 |
| Lidarex Com | 1.7307 | 0.5180 | 0.0573 |
| Scopolamine MZL | 0.3 | 21.13 | 0.63 |
| Oxymetazoline MZL | 0.5 | 0.35 | 0.0035 |
| Oxymethazoline Com | 0.5 | 0.33 | 0.0031 |
| Bupivicaine HCl MZL | 0.5 | 253.68 | 1.58 |
| Bupivicaine HCl Com | 0.5 | 226.75 | 1.35 |

The Release Rate was calculated as follows:

1. μg Active Released at time (min.)

$$= \frac{\text{Asam} \times \text{Mass Std} \times \text{VolReceptor} \times \text{Mass of Active Applied for Z cells} \times \text{C}}{\text{Astd} \times \text{Vol Std} \times \text{Label Claim} \times \text{Mass of Product Applied for 1 cell} \times \text{Z} \times 100}$$

WHERE:

| | |
|---|---|
| Asam | = Area of peak of sample solution |
| Astd | = Area of peak of standard solution |
| Mass Std | = Mass of standard taken to prepare the standard solution expressed in μg |
| Vol Std | = Volume to which the standard solution is made up, expressed in ml |
| Vol Receptor | = Volume of diffusion cells system receptor chamber, expressed in ml |
| Label Claim | = Amount of active present per 100g of product |
| Mass of Product Applied for 1 cell | = Specific amount of product applied for a specific cell |
| Mass of Active Applied for Z cells | = Amount of active applied in total for all Z cells utilised per one study |
| C | = potency of the standard, expressed as a percentage |

2.

Accumulative Dose (μg) released / square cm at time (min)

$$= \frac{\text{μg Active Released}}{\text{(Surface Area of Exposed Skin)}}$$

$$= \frac{\text{μg Active Released}}{1.767\text{cm}^2}$$

3.

Release Rate

$$= \frac{\text{Accumulative Dose (μg) released / square cm}}{\text{Time (hours)}}$$

4.

Percentage of Active Released at time (min.)

$$= \frac{\text{μg Active Released} \times 100}{\text{μg Active Applied}}$$

It was established that

A.  Doxipex Cream releases Doxepin hydrochloride 2.4754 times faster than the commercially available Doxepin Com product;

B.  Lidarex releases Lidocaine HCl 1.3425 times faster than the commercially available Lidocaine Com product.

D.  C. The release of Oxymetazoline HCLI from the MZL formulation compared well with that of the commercial Oxymetazoline Com formulation, but both values were very low, due to the thickness of the mucous membrane of the pig. The human mucous membrane is much thinner and release should accordingly be much higher in humans. The release of Bupivicaine from the MZL formulation was nevertheless shown to be 10.1% faster than that of the commercially available Bupivicaine formulation after 5 min and was about equal at all other times.

8. **CONCLUSION**

It is evident from the results obtained that the percentage active ingredient released from the DOXIPEX CREAM is significantly higher than that of the comparator Doxepin Com Cream. The release of Doxepin Hydrochloride from both the products consequently results in the effective administration of approximately the same quantity of active per dose although Doxipex contains 2% m/m Doxepin Hydrochloride and Doxepin Com 5% m/m Doxepin Hydrochloride in their respective formulations.

A comparison of the release rates at 60 minutes indicate that the release of Doxepin Hydrochloride from Doxipex Cream (2%) is at a rate 2.475 times greater than that from Doxepin Com Cream (5%). The percentage of active released from the formulation of the nanolipid vesicle formulation of Preparation 2 for this specific time is also 2.47 times greater than for the comparator.

**Example 3**

**CLINICAL STUDIES:**

Below is described three examples of the use of the Doxepin MZL Formulation in three patients presenting with pain due to three different clinical causes.

**CASE STUDY 1. USE OF DOXEPIN FORMULATION IN MANAGEMENT OF LOWER BACK PAIN AND DEPRESSION**

The doxepin in nanolipid formulation described above (DOXIPEX) was administered to the lower back area of a post-menopausal Caucasian woman suffering from manic depression associated with severe lower backache. Her depression was at the time reasonably under control through the use of PROZAC. She experienced substantial relief from the use of the formulation of the invention and discontinued her use of PROZAC after about 6 months on DOXIPEX. This is a most surprising result in view of the very low dose of doxepin hydrochloride administered through the topical application of the formulation of the invention.

**CASE STUDY 2. USE OF DOXEPIN FORMULATION IN MANAGEMENT OF PAIN IN REITER'S SYNDROME**

The doxepin in nanolipid formulation described above (DOXIPEX) was administered to the affected limbs of an active post-menopausal Caucasian woman diagnosed four years previously with Reiter's syndrome.

**Clinical symptoms in volunteer:**

Incapacitated and wasted right arm of the subject due to non-movement as a result of pain in the limb. The arm was carried in a light cast.

**Previous treatment of the subject:**

The subject was using intramuscular and /or oral pain relievers and muscle relaxants on a chronic basis since her diagnosis.

**Dosage form and usage of the MZL doxipin formula:**

MZL Doxipin was applied three times daily to the affected areas.

**Progress:**

After one month of usage, the subject no longer needed the support of the armcast. Muscle degeneration due to non-usage was reversed to some extent.

After 4 months of usage, the subject moved and used the arm freely. The wrist remained stiff.

**CASE STUDY 3: USE OF DOXEPIN FORMULATION IN MANAGEMENT OF SEVERE BACK PAIN IN A DEGENERATIVE THORACIC AND VERTEBRAL CONDITION.**

**Subject:**

The subject was an 80 year old Caucasian male with severe back pain due to degeneration of his vertebrae. He was nearly completely immobilised by his condition and seldom moved outside of his home. He was on chronic therapy of intramuscular pain relievers and muscle relaxants.

**Dosage form and usage of the MZL Doxepin formula:**

In his case, synthetic polymer cutaneous patches were saturated with MZL Doxepin Formulation and attached at night to areas where he experienced extreme pain for prolonged release of the active compound.

**Progress:**

After a year of treatment, the subject was completely mobile. He stopped taking medication for pain, either of commercial origin or the Doxepin Formulation.

**comparative example 4**

**DISSOLUTION STUDIES IN RESPECT OF FORMULATIONS MADE ACCORDING TO THE INVENTION**

Formulations of the active agents referred to below were prepared in the manner described above and were subjected to dissolution tests (2 or 3 repeats as indicated below) performed in terms of the USP 24 procedure. The following results were obtained.

| Product | Limits | Results |
| --- | --- | --- |
| Carbamazipine MZL suspension | NTL 75% in 30 min | 123%, 112%, 114% |
| Carbamazipine Com suspension | NTL 75% in 30 min | 111%, 112%, 112% |
| Zolpidem Tartrate MZL SGC | NTL 75% | 92%, 98%, ND |
| Zolpidem Tartrate Com SGC | NTL 75% | 102% 102% |
| Propranolol MZL SGC | NTL 75% in 30 min | 124% 126% 130% |
| Propranolol Com SGC | NTL 75% in 30 min | 106% 105% 107% |
| Phenythion Na MZL SGC | NTL 85% in 30 min | 143% 147% 155% |
| Phenythion Na Com SGC | NTL 85% in 30 min | 53%, 46%, 46% |

The advantages of the MZL formulations over the commercial formulations are self-evident.

**Claims**

1.   A pharmaceutical preparation comprising Doxepin formulated with an administration medium which Is **characterised in that** it comprises a solution of nitrous oxide gas in a pharmaceutically acceptable carrier solvent for the gas and which administration medium includes at least one fatty acid or ester or other suitable derivative thereof selected from the group consisting of oleic acid, linoleic acid, alpha-linolenic acid, gamma-linolenic acid, arachidonic acid, eicosapentaenoic acid [C20: 5ω3], decosahexaenoic acid [C22: 6ω3], ricinoleic acid and derivatives thereof selected from the group consisting of the C1 to C6 alkyl esters thereof, the glycerol-polyethylene glycol esters thereof and the reaction product of hydrogenated natural oils composed largely of ricinoleic acid based oils such as castor oil with ethylene oxide.

2.   The preparation of claim 1 wherein the essential fatty acid or ester thereof, component of the composition comprises a mixture of esters of the fatty acids listed above and is preferably constituted by the complex known as Vitamin F Ethyl Ester having a typical fatty acid distribution as follows:

$< C_{16}$: 0
$C_{16.0}$ : 8,3 %
$C_{18.0}$ : 3,5 %
$C_{18\cdot 1}$ : 21,7 %
$C_{18.2}$ : 34,8 %
$C_{18.4}$ : 28,0 %
$> C_{18}$: 1,6 %
unknown: 2,1 %.

3. The preparation of claim 2 wherein the administration medium further includes eicosapentaenoic acid [C20: 5ω3] and/or decosahexaenoic acid [C22: 6ω3] as additional long chain fatty adds.

4. The preparation of claim 1 wherein the carrier solvent is water (preferably delonised water) or any of the pharmaceutically acceptable alcohols, ethers, polymers such as a polyethyleneglycol or the like or an oil which is preferably an organic oil which organic oil is further preferably an essential oil based on long chain fatty acids having between 14 and 22 carbon atoms in the fatty acid and is preferably of natural origin and most preferably a plant oil rich in gamma linolenic acid [GLA].

5. The preparation of claim 1 wherein the Doxepin is formulated in a liquid presentation and wherein the formulation incorporate, as part of the administration medium, water or acceptable other liquid solvent into which the nitrous oxide is dissolved, preferably to saturation, and wherein the fatty acid or ester thereof is dissolved or suspended or emulsified along with the Doxepin.

6. The preparation of claim 1 wherein the Doxepin is formulated to be applied as a topical, buccal or vaginal cream or ointment or as a cutaneous patch, or as an intravenous, intramuscular or subcutaneous injection, or as a suppository, and wherein the formulation used in making up such cream, ointment, injectable formulation or suppository incorporates, along with the Doxepin to be enhanced, a quantity of water containing, and preferably saturated with, nitrous oxide, the long chain fatty acid or ester thereof and, optionally further incorporates such additional excipients and carriers as are conventionally used in the pharmaceutical trade in making up such dosage forms.

**Patentansprüche**

1. Pharmazeutisches Präparat, das mit einem Verabreichungsmedium formuliertes Doxepin beinhaltet, **dadurch gekennzeichnet, dass** es eine Lösung von Distickstoffoxidgas in einem pharmazeutisch akzeptablen Trägerlösungsmittel für das Gas beinhaltet, wobei das Verabreichungsmedium wenigstens eine Fettsäure oder einen Ester oder ein anderes geeignetes Derivat davon beinhaltet, ausgewählt aus der Gruppe bestehend aus Ölsäure, Linolsäure, Alpha-Linolensäure, Gamma-Linolensäure, Arachidonsäure, Eicosapentaensäure [C20: 5ω3], Decosahexaensäure [C22: 6ω3], Ricinolsäure und Derivaten davon, ausgewählt aus der Gruppe bestehend aus den C1 bis C6 Alkylestern davon, den Glycerolpolyethylenglykolestern davon und dem Reaktionsprodukt gehärteter natürlicher Öle, die größtenteils aus Ricinolsäure-basierten Ölen bestehen, wie Rizinusöl mit Ethylenoxid.

2. Präparat nach Anspruch 1, wobei die Komponente essentielle Fettsäure oder Ester davon der Zusammensetzung ein Gemisch aus Estern der oben aufgelisteten Fettsäuren beinhaltet und vorzugsweise aus dem als Vitamin F Ethylester bekannten Komplex zusammengesetzt ist, mit der folgenden typischen Fettsäureverteilung:

$< C_{16}$: 0
$C_{16,0}$: 8,3 %
$C_{18,0}$: 3,5 %
$C_{18,1}$: 21,7 %
$C_{18,2}$: 34,8 %
$C_{18,\,4}$ 28,0 %
$>C_{18}$: 1,6 %
unbekannt: 2,1 %.

3. Präparat nach Anspruch 2, wobei das Verabreichungsmedium ferner Eicosapentaensäure [C20: 5ω3] und/oder Decosahexaensäure [C22: 6ω3] als zusätzliche langkettige Fettsäuren beinhaltet.

4. Präparat nach Anspruch 1, wobei das Trägerlösungsmittel Wasser (vorzugsweise deionisiertes Wasser) oder ein beliebiger/s der pharmazeutisch akzeptablen Alkohole, Ether, Polymere wie Polyethylenglykol oder dergleichen oder ein Öl ist, das vorzugsweise ein organisches Öl ist, das ferner vorzugsweise ein ätherisches Öl auf der Basis von langkettigen Fettsäuren mit 14 bis 22 Kohlenstoffatomen in der Fettsäure ist und vorzugsweise natürlichen Ursprungs und am bevorzugtesten ein an Gamma-Linolensäure [GLA] reiches Pflanzenöl ist.

5. Präparat nach Anspruch 1, wobei das Doxepin in einer flüssigen Darreichung formuliert ist und wobei die Formulierung als Teil des Verabreichungsmediums Wasser oder ein akzeptables anderes flüssiges Lösungsmittel beinhaltet, in dem das Distickstoffoxid gelöst ist, vorzugsweise bis zur Sättigung, und wobei die Fettsäure oder der Ester davon zusammen mit dem Doxepin gelöst oder suspendiert oder emulgiert wird.

6. Präparat nach Anspruch 1, wobei das Doxepin zur Anwendung als topische, bukkale oder vaginale Creme oder Salbe oder als kutanes Pflaster oder als intravenöse, intramuskuläre oder subkutane Injektion oder als Suppositorium formuliert ist, wobei die zur Herstellung einer solchen Creme, Salbe, injizierbaren Formulierung oder eines solchen Suppositoriums verwendete Formulierung zusammen mit dem aufzuwertenden Doxepin eine Wassermenge, die Distickstoffoxid enthält und vorzugsweise damit gesättigt ist, die langkettige Fettsäure oder den Ester davon und optional des Weiteren zusätzliche Exzipienten und Träger enthält, die in der Pharmazie gewöhnlich zur Herstellung solcher Dosierungsformen verwendet werden.

## Revendications

1. Préparation pharmaceutique comprenant de la doxépine formulée avec un milieu d'administration qui est **caractérisé en ce qu'**il comprend une solution de gaz d'oxyde nitreux dans un solvant porteur pharmaceutiquement acceptable pour le gaz et lequel milieu d'administration comprend au moins un acide gras ou un ester ou autre dérivé approprié de celui-ci sélectionné parmi le groupe consistant en acide oléique, acide linoléique, acide alpha-linolénique, acide gamma-linolénique, acide arachidonique, acide éicosapentaénoïque [C20: 5ω3], acide docosahexaénoïque [C22: 6ω3], acide ricinoléique et des dérivés de ceux-ci sélectionnés parmi le groupe consistant en les esters d'alkyle en C1 à C6 de ceux-ci, les esters de glycérol-polyéthylène glycol de ceux-ci et le produit de réaction d'huiles naturelles hydrogénées composées en grande partie d'huiles à base d'acide ricinoléique telles que de l'huile de ricin avec de l'oxyde d'éthylène.

2. Préparation selon la revendication 1, dans laquelle l'acide gras essentiel ou ester de celui-ci, composant de la composition comprend un mélange d'esters des acides gras énumérés ci-dessus et est constitué de préférence par le complexe connu comme de l'ester éthylique de vitamine F ayant une distribution d'acides gras typique comme suit:

$<C_{16}$:0
$C_{16.0}$ : 8,3 %
$C_{18.0}$ : 3,5 %
$C_{18.1}$ : 21,7 %
$C_{18.2}$ : 34,8 %
$C_{18.4}$ : 28,0 %
$> C_{18}$ : 1,6 %
inconnu : 2,1 %

3. Préparation selon la revendication 2, dans laquelle le milieu d'administration comprend en outre de l'acide éicosapentaénoïque [C20: 5ω3] et/ou de l'acide docosahexaénoïque [C22: 6ω3] en tant qu'acides gras à chaîne longue supplémentaires.

4. Préparation selon la revendication 1, dans laquelle le solvant porteur est de l'eau (de préférence de l'eau déminéralisée) ou bien l'un quelconque des alcools, éthers, polymères pharmaceutiquement acceptables tels qu'un polyéthylène glycol ou similaire ou une huile qui est de préférence une huile organique, laquelle huile organique est en outre de préférence une huile essentielle basée sur des acides gras à chaîne longue ayant d'entre 14 et 22 atomes de carbone dans l'acide gras et est de préférence d'origine naturelle et le plus préférentiellement une huile végétale riche en acide gamma-linolénique [GLA].

**5.** Préparation selon la revendication 1, dans laquelle la doxépine est formulée dans une préparation liquide et dans laquelle la formulation incorpore, comme faisant partie du milieu d'administration, de l'eau ou un autre solvant liquide acceptable dans lequel l'oxyde nitreux est dissous, de préférence à saturation, et dans laquelle l'acide gras ou l'ester de celui-ci est dissous ou mis en suspension ou émulsionné avec la doxépine.

**6.** Préparation selon la revendication 1, dans laquelle la doxépine est formulée pour être appliquée comme une crème ou une pommade topique, buccale ou vaginale, ou bien comme un timbre cutané, ou bien comme une injection intraveineuse, intramusculaire ou sous-cutanée, ou bien comme un suppositoire, et dans laquelle la formulation utilisée dans la fabrication d'une telle crème, pommade, formulation injectable ou suppositoire, incorpore, avec la doxépine à mettre en valeur, une quantité d'eau contenant et de préférence saturée avec de l'oxyde nitreux, l'acide à chaîne longue ou ester de celui-ci et incorpore optionnellement en outre de tels excipients et véhicules additionnels tels qu'ils sont utilisés conventionnellement dans le commerce pharmaceutique pour fabriquer de telles formes d'administration.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 939128773 A
- US 5633284 A
- WO 9717978 A
- US 6221377 B

- GB 1033299 A
- GB 1105919 A
- EP 0123827 A

**Non-patent literature cited in the description**

- Monthly Index of Medical Specialities (''MIMS). Times Media in South Africa

- Goodman & Gilman's The Pharmacological Basis of Therapeutics. 1990, 298-300
- The Merck Index. 6499